(19) Europäisches Patentamt
European Patent Office
Office européen des brevets

(11) Publication number: 0 069 512
B1

(12) EUROPEAN PATENT SPECIFICATION

(45) Date of publication of patent specification: 04.06.86

(51) Int. Cl.[4]: A 61 K 7/00, A 61 K 9/00, A 61 K 47/00, C 07 D 207/28

(21) Application number: 82303312.1

(22) Date of filing: 25.06.82

# (54) Salts of N-substituted-2-pyrrolidone-4-carboxylic acids as humectants.

(30) Priority: 08.07.81 US 281445

(43) Date of publication of application:
12.01.83 Bulletin 83/02

(45) Publication of the grant of the patent:
04.06.86 Bulletin 86/23

(84) Designated Contracting States:
AT BE CH DE FR GB IT LI LU NL SE

(56) References cited:
FR-A-1 348 617
GB-A-1 323 061
GB-A-2 040 998
US-A-2 757 125
US-A-3 803 141

THE JOURNAL OF ORGANIC CHEMISTRY, vol. 45, no. 5, 29th February 1980, pages 810-814, American Chemical Society, Easton, P.A., USA P.A. ZORETIC et al.: "Synthetic approaches to 10-azaprostaglandins"

(73) Proprietor: PFIZER INC.
235 East 42nd Street
New York, N.Y. 10017 (US)

(72) Inventor: Sysko, Robert J.
6 Stone Cliffe Drive
Niantic New London Connecticut (US)
Inventor: Tate, Bryce Eugene
11 Laurel Hill Drive
Niantic New London Connecticut (US)

(74) Representative: Wood, David John et al
Pfizer Limited Ramsgate Road
Sandwich Kent CT13 9NJ (GB)

EP 0 069 512 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Alkali metal salts of 2-pyrrolidone-4-carboxylic acid derivatives of the formula

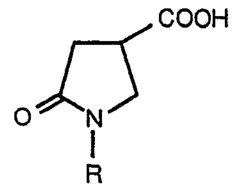

(I)

wherein R is ($C_1$—$C_5$)alkyl or ($C_1$—$C_5$)hydroxyalkyl (with the proviso that the hydroxy group is not on a carbon adjacent to the nitrogen atom), are useful as humectants, particularly in topical preparations for use with mammals.

The acid forms of the N-alkyl variants of the present invention have been previously reported as useful in functional fluids such as hydraulic fluids, acid pickling and cleaning compositions, and glycol antifreeze compositions as metal corrosion inhibitors, particularly for aluminum, copper and brass (British Patent 1,323,061). The higher homologs, *viz.,* the N-($C_{12}$—$C_{20}$)alkyl derivatives, in free acid or salt form, have been reported as ashless, antirust lubricating oil additives (Hinkamp, U.S. Patent 3,224,975), and as antibacterial additives in dental creams, mouthwashes and shampoos. It will be noted that such higher homologs are essentially lacking in the desirable hygroscopic properties of the present humectant compounds.

The alkyl esters of N-alkyl-2-pyrrolidone-4-carboxylic acids have been variously reported as synthetic intermediates useful as intermediates in the preparation of a variety of pharmaceutically useful compounds (U.S. Patent 3,803,141; Japan 51-032,555; Belgium 835,317; Germany 2,452,536).

The acid forms of the N-hydroxyalkyl variants of the present invention are disclosed as intermediates in the preparation of certain ester monomers, which in turn are useful in the preparation of polyester lubricant additives (Elliott *et al.,* U.S. Patent 4,127,493).

The hygroscopic salts of structurally related 2-pyrrolidone-5-carboxylic acid and its 1-methyl and 4-methyl analogs:

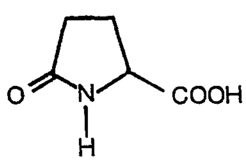 , 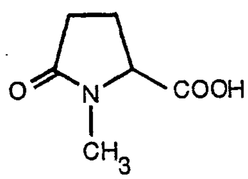 and 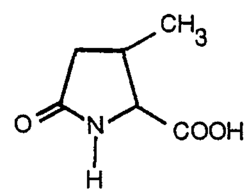

are reported to have humectant activity (Laden, U.S. Patent 3,235,457). In fact, the salts of 2-pyrrolidone-5-carboxylic acid itself are reported as naturally occurring moisturizing agents in skin [Laden and Spitzer, J. Soc. Cosmet. Chem. 18, pp. 351—360 (1967)], and sodium 2-pyrrolidone-5-carboxylate is a commercially available humectant sold, for example, under the tradename "Ajidew N—50" by Ajinomoto Co., Inc., Tokyo, Japan. The present compounds, however, show humectant activity surprisingly superior to this commercial product. In particular, it will be noted that the isomeric compound most closely related to the present compounds, *viz.,* the sodium salt of 2-pyrrolidone-4-carboxylic acid is essentially devoid of the desirable hygroscopic properties of the present humectant compounds. More generally, alkali metal or ammonium salts of acids of the formula

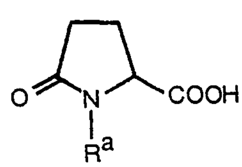

wherein $R^a$ is hydrogen or ($C_1$—$C_4$)alkyl are reported to be useful as conditioners in shampoo preparations (Japanese Patent Document 74-027,643).

The present invention is concerned with humectant compounds of the formula

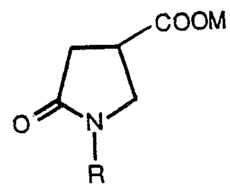

(II)

wherein M is an alkali metal (preferably Na or K and most preferably Na) and R is ($C_1$—$C_5$)alkyl or ($C_1$—$C_5$)hydroxyalkyl with the proviso that the hydroxy group is not substituted on the carbon atom adjacent to the nitrogen atom. These compounds find use as humectants in such diverse products as tobacco, printing inks, synthetic films and fibers, paper products and paints, but, because of their unusually high hygroscopicity and low toxicity, they are particularly valuable for use in topical, water-containing preparations with mammals. Exemplary of such preparations, requiring protection from loss of water, particularly after topical application, are cosmetics (lotions, face creams, make-up preparations, perfumes, hair preparations, medicated cosmetics); detergent compositions (soaps, bath preparations, shampoos, dentifrices), and pharmaceutical preparations (ointments, oils, liniments, suppositories).

Such cosmetic, detergent and pharmaceutical preparations for topical use in mammals comprise water and from 3 to 30% by weight of the alkali metal salt of the formula (II). Even 50% water solutions of these salts are not toxic.

Levels in the narrower range of about 5 to 15% are generally preferred, since usually such levels sufficiently minimize water loss from the preparation.

The compounds required for the present humectant use are readily prepared by heating about 1 to 1.1 molar equivalents of the appropriate primary amine with itaconic acid:

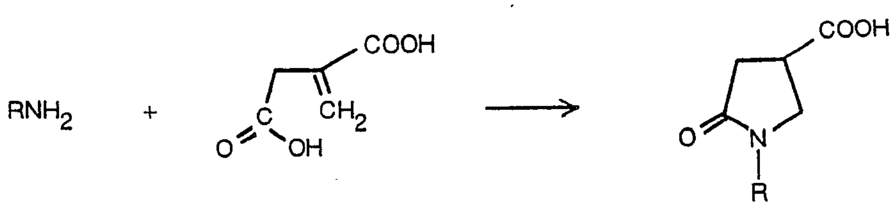

wherein R is as defined above. The reaction can be carried out neat or in the presence of a reaction-inert diluent, conveniently, and least expensively, water. Temperature is not critical, but is generally in the range of about 80—150°C., preferably in the range 95—135°C., sufficiently high that the reaction proceeds at a reasonable rate, but not so high as to cause undue decomposition of the starting materials or product. In the preferred temperature range, the reaction is generally carried out in an autoclave when a volatile amine and/or solvent is present. The desired salt is preferably formed directly in the reaction mixture by neutralization with the appropriate alkali metal base, e.g. sodium hydroxide, sodium carbonate, potassium hydroxide. Any excess amine is stripped from the reaction mixture and the reaction stripped of solvent, if present. Alternatively, when water is employed as solvent, the reaction mixture is concentrated to the desired concentration of product in water, up to about 50% w/w (0.5 g./g.). Such a concentrated solution is generally as well-suited as the anhydrous salt for the present preferred use, *viz.,* water-containing preparations for topical use with mammals.

The humectant properties of the compounds of the formula (II), reflecting their present utility, are determined by equilibrium hygroscopicity measurements with the isolated salts, and by measuring moisture retention in a typical preparation for topical use with mammals. The latter testing is carried out according to the method of Griffin *et al.,* J. Soc. Cosmetic Chem. 3, p. 5 (1952) using a hand cream formulated according to specific Example 7 below. Typical hygroscopicity data are shown in Table I, while moisture retention measurements are shown in Table II. The hygroscopicity data (Table I) are most pertinent to the present preferred use with mammals, since these data reflect the ability of the compounds to retain maximum moisture at equilibrium, after topical application has occurred.

TABLE I

Equilibrium Weight Increase (%) of Humectant Compounds under Specific Conditions of Relative Humidity

| Compound | R | % Weight Increase at Relative Humidity | | |
|---|---|---|---|---|
| | | 31% | 53% | 83% |
| 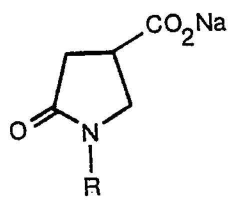 | H– | – | 2 | – |
| | $CH_3$– | 1 | 65 | 130 |
| | $n$-$C_4H_9$– | 14 | 41 | – |
| | $HOCH_2CH_2$– | 4 | 38 | – |
| | $n$-$C_{12}H_{25}$– | 3 | 9 | – |
| 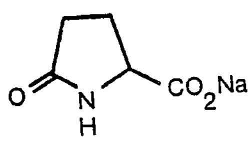 | | 6 | 33 | 85 |
| Sorbitol | | 1 | 2 | 42 |
| Glycerin | | 6 | 29 | – |
| Propylene glycol | | 5 | 9 | 50 |

TABLE II

Moisture Loss (%) from a Formulated Hand Cream Cast as a Thin Film over Sand under Constant Relative Humidity of 83%

| Compound | Moisture Loss (%) in 45 Hours at Relative Humidity | |
|---|---|---|
| | 53% | 83% |
| Control | 28 | 13 |
| Sorbitol | - | 12 |
| Glycerin | - | 12 |
| 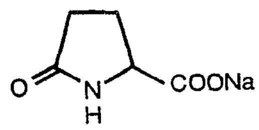 | 24 | 10 |
| 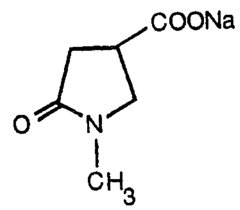 | 24 | 9 |
| 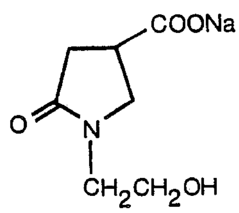 | 25 | - |

The present compounds are particularly valuable humectants in cosmetic, detergent and pharmaceutical preparations because they are essentially non-toxic under the conditions of use. Thus neither sodium N-methyl-2-pyrrolidone-4-carboxylate nor sodium N-(2-hydroxyethyl)-2-pyrrolidone-4-carboxylate at an oral dose of 5 g./kg. in rats showed any mortality over a 14 day observation period. The only drug induced effects noted at this high dose was slight salivation within minutes of dosing and diarrhea which persisted for 24 hours. Single topical doses of 2 g./kg. of the same compounds (as 0.5 g./g. solutions in water) applied to the normal or abraded back skin of rabbits likewise produced no mortality in the course of the 2 week observation period. Although a well-defiuned erythema was apparent on the backs of animals with abraded back skin at 24 hours, these backs were normal at 48 hours. No adverse effects were noted with normal skin. Instillation of 0.1 ml. of 0.5 g./g. solutions of the same compounds into the conjunctival sacs of the eyes of albino rabbits did not produce a positive irritating effect. Reactions produced in rinsed or unrinsed eyes by either solution were minimal and transient in nature, the eyes generally returning to normal within 24 hours of dosing.

The compounds are incorporated into standard water-containing cosmetic, detergent and pharmaceutical preparations according to methods well known in the cosmeticians and pharmacists art.

The present invention is illustrated by the following examples. However, it should be understood that the invention is not limited to the specific details of these examples.

Example 1

N-Methyl-2-pyrrolidone-4-carboxylic Acid

Method A

To a flask equipped with a rubber septum, dry ice/acetone condenser, magnetic stirrer, and heating mantle, was charged 26.02 g. (200 mmol) of itaconic acid. Methylamine (17.14 g., 220 mmol, of 40% aqueous solution) was added slowly by syringe. After the initial exotherm had subsided, the mixture was heated under reflux with stirring for 11.5 hours. The solution was diluted with water and treated with excess Amberlite 200 to neutralize any amine salt present. The resin was removed by filtation and the filtrate was evaporated to dryness, triturated with ether, and vacuum dried to yield 26.68 g. (93%) of crude product. Recrystallization from ethyl acetate gave 24.23 g. (85%) of pure product as colorless needles, m.p. 151—153° (lit. m.p. 153—154°); ir (KBr) 1720 (acid C=0) and 1625 $cm^{-1}$ (amide C=0); nmr ($D_2O$) delta 4.07—2.93 (m, 3, —N—$C\underline{H}_2$— and —C$\underline{H}$—), 2.87 (s, 3, —N—$C\underline{H}_3$), 2.74 (d[AB], 2, *J*=8 Hz, O=C—$C\underline{H}_2$—).

Alternatively at the end of the 11.5 hour reflux period, the reaction mixture was cooled to room temperature and 18.2 g. (64%) of product isolated directly by filtration. By evaporation of the filtrate and recrystallization of the residue from acetone there was obtained 6.3 g. (22%) of additional product.

The reaction was repeated employing only 1.0 equivalent (15.58 g., 200 mmole) of the 40% aqueous solution of methylamine. By the second isolation method there was obtained 21.7 g. (76%) directly from the cooled reaction mixture and an additional 2.3 g. (8%) from filtrate.

Method B

A stainless steel bomb was charged with a slurry of 26.02 g. (0.2 mole) of itaconic acid and 10.3 ml. of water. Methylamine (17.14 g., 0.22 mole of a 40% aqueous solution) was added. An initial exotherm to 55—60°C., due to salt formation, was noted. The bomb was sealed and heated to 110°C., a pressure of 21 psig being noted. At the end of 10 hours, the bomb was cooled and product isolated according to one of the alternatives of method A immediately above. Alternatively, the product acid is converted to sodium salt *in situ* by the addition of no more than one equivalent of sodium hydroxide, excess amine is removed by stripping, and sodium salt or salt solution isolated according to one of the methods of the following example.

Example 2

Sodium N-Methyl-2-pyrrolidone-4-carboxylate

Method A (dry solids)

N-Methyl-2-pyrrolidone-4-carboxylic acid (5.00 g., 0.03876 mole) was dissolved with warming and stirring in 25 ml. of deionized water. The solution was cooled to room temperature, and 1.00 N sodium hydroxide (38.70 ml., slightly under 1 equivalent) added slowly. Evaporation and drying the residue to constant weight at 40—50°C. in a vacuum oven gave title product in quantitative yield.

Method B (50% w/w aqueous solution)

Itaconic acid (390 g., 3.0 moles), 25% w/w methylamine in water (410 g., 3.3 mole) were combined and gently stirred in an ice-water bath until the exotherm had subsided. During this process the temperature rose to 70°C. and then fell to 30°C. The resulting thick mass was then heated to reflux (internal temperature 105°C.) for 25 hours. The reaction mixture was diluted with 925.5 ml. of water, than the pH adjusted to a stable pH of 8.00—8.02 with 4.8 N sodium hydroxide (total volume of 611.2 ml., 98% of theory). The solution was heated and treated with activated carbon, filtered with water wash, and the combined filtrate and wash evaporated to a weight of 970 g. containing 0.478 g. of title product per gram of aqueous solution, i.e. a 47.8% w/w solution.

An aliquot was evaporated to the free acid form, prior to acidification. It showed $^1H$—NMR in $D_2O$: delta (ppm) 3.92—2.92 [m, 3H, $NC\underline{H}_2$ and C$\underline{H}$(COOH)], 2.85 (s, 3H, $NC\underline{H}_3$), 2.65 (d, 2H, J=7 Hz, $COC\underline{H}_2$).

Example 3

N-(2-Hydroxyethyl)-2-pyrrolidone-4-carboxylic Acid

To a flask equipped for distillation was charged 65.00 g. (500 mmol) of itaconic acid and 33.60 g. (550 mmol) of distilled ethanolamine. The mixture was stirred magnetically, heated at 130°, and the water formed during the reaction was collected by distillation. After heating for 3—4 hours, residual water was removed on the rotary evaporator to yield 85.00 g. (98%) of product as an extremely viscous amber syrup;

ir (neat) 3350 (OH), 1730 (acid C=O), and 1660 $cm^{-1}$ (amide C=O); $^1$H—NMR ($D_2O$) delta 4.00—3.57 (m, 4, —C<u>H</u>$_2$ and N—C<u>H</u>$_2$ [ring]), 3.57—3.00 (m, 3, C<u>H</u> and N—C<u>H</u>$_2$— [exo]), 3.28 (d[AB], 2, *J*=7 Hz, O=C—C<u>H</u>$_2$).

Example 4
Sodium N-(2-Hydroxyethyl)-2-pyrrolidone-4-carboxylate

Itaconic acid (390 g., 3.0 mole) and 2-hydroxyethylamine (201.3 g., 3.3 mol) were reacted neat, with ice-water bath cooling to control the exotherm noted on mixing. Following subsiding of the exotherm, the reaction was heated at 130°C. for 6.25 hours, collecting 49.2 g. of water (91% of theory) as distillate. Following the procedure of method B of Example 2, the reaction mixture was converted to 1170 g. of 49.4 g./g. (49.4% w/w) aqueous solution of title product. $^1$H—NMR ($D_2O$) delta: 3.90—2.80 (m, 7H, C<u>H</u>$_2$O, NC<u>H</u>$_2$ [ring], C<u>H</u>$CO_2$Na, NC<u>H</u>$_2$ [exo], 2.63 (d, 2H, J=10 Hz, COC<u>H</u>$_2$).

Example 5
N-(1-Butyl)-2-pyrrolidone-4-carboxylic Acid

A mixture of 26.02 g. (200 mmol) of itaconic acid and 16.13 g. (220 mmole) of *n*-butylamine was refluxed with stirring for 6 hours. Water and excess amine were removed on the rotary evaporator yielding 36.70 g. (99%) of title product as a nondistillable syrup. A portion of this product was heated under vacuum to remove any traces of volatile impurities; ir (neat) 1730 (acid C=O) and 1640 $cm^{-1}$ (amide C=O); nmr ($CDCl_3$) delta 11.77 (s [exchanged with $D_2O$], 1, —$CO_2$<u>H</u>), 3.67 (m, 2, N—C<u>H</u>$_2$ [ring]), 3.32 (m, 3, C<u>H</u>COOH and NC<u>H</u>$_2$ [exo]), 2.80 (m, 2, OC—C<u>H</u>$_2$), 1.45 (m, 4, *n*-butyl-C<u>H</u>$_2$—), 0.97 (m, 3, —C<u>H</u>$_3$).

| | | | |
|---|---|---|---|
| *Anal.* Calcd. for $C_9H_{15}NO_3$: | C, 58.36; | H, 8.16; | N, 7.56. |
| Found: | C, 58.56; | H, 8.01; | N, 7.96. |

Example 6
Sodium N-(1-Butyl)-2-pyrrolidone-4-carboxylate

Free acid of the preceding example (10.347 g., 55.9 mmole) was combined with 25 ml. of water. The pH was adjusted to 8.90 using approximately 1 equivalent of aqueous NaOH (4.8 N), and the mixture heated, treated with activated carbon, filtered and evaporated to a thick oil which solidified on vacuum drying. Yield 9.71 g.

Example 7
<u>Handcream Formulations</u>

The following ingredients were combined in parts by weight and heated to 80°C.:

| | |
|---|---|
| Mineral oil | 2.7 |
| Paraffin | 0.3 |
| Petrolatum | 0.4 |
| Isopropyl myristate | 0.5 |
| Beeswax | 0.5 |
| Cetyl alcohol | 0.1 |
| Sorbitan monostearate | 0.16 |
| Polyoxyethylene lanolin derivative | 0.34 |

Separately, the sodium salt of example 2, 4 or 6 was combined with water to form a solution 0.5 parts by weight of the sodium salt and 4.5 parts by weight of water. This solution was warmed to 80°C. and added to the above mixture at 80°C.

The mixture was then cooled to 40°C., divided in the desired amount into the desired receptacles, and cooled to ambient temperature.

When these formulations were used for moisture retention studies the receptacles were 7 dram vials. One gram of hand cream was combined with one gram of sea sand and tested for water retention according to the method of Griffin *et al.,* J. Soc. Cosmetic Chem. 3, p. 5 (1952).

Example 8
Alternative Handcream Formulation

The following ingredients were combined and heated to 70°C.:

| | |
|---|---|
| Mineral oil | 26.3 g. |
| Beeswax | 5.0 g. |
| Fatty glycerides | 17.5 g. |
| Sorbitan monostearate | 3.0 g. |
| Polyoxyethylated sorbitan monostearate | 4.0 g. |

Cellulose gum (0.2 g.) was dissolved in water. Aqueous sodium N-methyl-2-pyrrolidone-4-carboxylate was added so as to form a mixture of

| | |
|---|---|
| Cellulose gum | 0.2 g. |
| Sodium salt | 5.0 g. |
| Water | 39.0 g. |

The resulting solution was heated to 72°C. and added slowly to the above mixture with good agitation. The mixture was cooled to 40°C. and filled into 4 oz. bottles. The capped bottles were labelled and stored at ambient temperature.

Example 9
Skin Lotion

The following ingredients are combined in parts by weight and heated to 80°C.:

| | |
|---|---|
| Stearic acid | 3.0 |
| Lanolin | 0.6 |
| Glyceryl monostearate | 1.0 |
| Mineral oil | 5.0 |

The following ingredients are combined in sequence at 80°C.:

| | |
|---|---|
| Water | 83.8 |
| Humectant | 5.0 |
| Magnesium-aluminum silicate complex | 0.75 |
| Triethanolamine | 0.78 |

The hot aqueous phase is added to the hot oil phase with thorough mixing. The mixture is cooled to 40°C. and filled into suitable receptacles, capped, labelled and stored at ambient temperature.

**Claims for the Contracting States: BE CH DE FR GB IT LI LU NL SE**

1. A method of minimizing water loss from a water-containing topical preparation for mammals which requires protection from said water loss, which comprises incorporating into said preparation from 3 to 30% by weight of an alkali metal salt of a compound of the formula

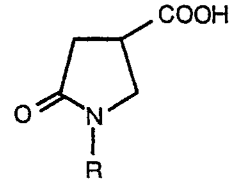

(I)

wherein R is ($C_1$—$C_5$)alkyl or ($C_1$—$C_5$)hydroxyalkyl, with the proviso that the hydroxy group is not on a carbon adjacent to the nitrogen atom.

2. A method as claimed in claim 1 wherein the preparation is a pharmaceutical or cosmetic preparation.

3. A method as claimed in claim 1 or 2 wherein the salt is a sodium salt.

4. A method as claimed in any one of the preceding claims wherein R is methyl, 1-butyl or 2-hydroxyethyl.

5. A water-containing topical preparation for mammals which comprises 3—30% by weight of an alkali metal salt of a compound of the formula

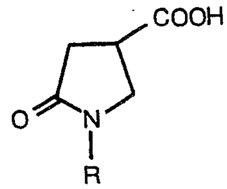

wherein R is ($C_1$—$C_5$)alkyl or ($C_1$—$C_5$)hydroxyalkyl, with the proviso that the hydroxy group is not substituted on the carbon adjacent to the nitrogen atom.

6. A preparation as claimed in claim 5 which is a pharmaceutical or cosmetic preparation.

7. A preparation as claimed in claim 5 or 6 wherein the salt is a sodium salt.

8. A preparation as claimed in any one of claims 5 to 7 wherein R is methyl, 1-butyl or 2-hydroxyethyl.

9. An alkali metal salt of a compound of the formula:

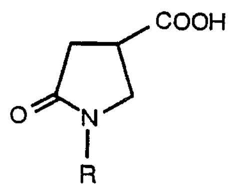

wherein R is ($C_1$—$C_5$)alkyl or ($C_1$—$C_5$)hydroxyalkyl with the proviso that the hydroxy group is not on a carbon adjacent to the nitrogen atom.

10. A salt as claimed in claim 9, which is a sodium salt.

**Claims for the Contracting State: AT**

1. A method of minimizing water loss from a water-containing topical preparation for mammals which requires protection from said water loss, which comprises incorporating into said preparation from 3 to 30% by weight of an alkali metal salt of a compound of the formula

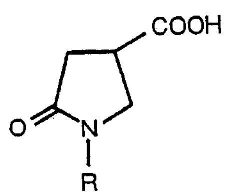

wherein R is ($C_1$—$C_5$)alkyl or ($C_1$—$C_5$)hydroxyalkyl, with the proviso that the hydroxy group is not on a carbon adjacent to the nitrogen atom.

2. A method as claimed in claim 1 wherein the preparation is a pharmaceutical or cosmetic preparation.

3. A method as claimed in claim 1 or 2 wherein the salt is a sodium salt.

4. A method as claimed in any one of the preceding claims wherein R is methyl, 1-butyl or 2-hydroxyethyl.

5. A process for preparing a water-containing topical preparation for mammals which comprises formulating an aqueous topical composition comprising 3—30% by weight of an alkali metal salt of a compound of the formula:

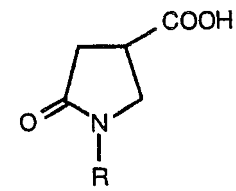

wherein R is ($C_1$—$C_5$)alkyl or ($C_1$—$C_5$)hydroxyalkyl, with the proviso that the hydroxy group is not substituted on the carbon adjacent to the nitrogen atom.

6. A process as claimed in claim 5 which comprises forming a pharmaceutical or cosmetic preparation.

7. A process as claimed in claims 5 or 6 wherein the salt is a sodium salt.

8. A process as claimed in any one of claims 5 to 7 wherein R is methyl, 1-butyl or 2-hydroxyethyl.

9. A process for preparing an alkali metal salt of a compound of the formula:

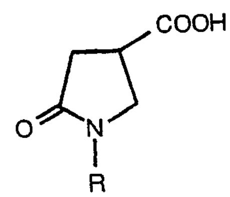

wherein R is ($C_1$—$C_5$)alkyl or ($C_1$—$C_5$)hydroxyalkyl with the proviso that the hydroxy group is not on a carbon adjacent to the nitrogen atom, which comprises reacting the corresponding free acid with an alkali metal base.

10. A process as claimed in claim 9, wherein the salt is a sodium salt.

**Patentansprüche für die Vertragsstaaten: BE CH DE FR GB IT LI LU NL SE**

1. Verfahren zum Minimieren des Wasserverlusts von einer Wasser-enthaltenden örtlich anwendbaren Präparation für Säugetiere, die Schutz gegenüber Wasserverlust benötigt, das das Einarbeiten in die Präparation von 3—30 Gew.% eines Alkalimetallsalzes einer Verbindung der Formel:

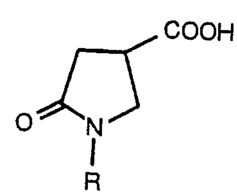

umfaßt, worin R ($C_1$—$C_5$)-Alkyl oder ($C_1$—$C_5$)-Hydroxyalkyl ist, mit der Maßgabe, daß die Hydroxygruppe nicht an einem zu dem Stickstoffatom benachbarten Kohlenstoffatom ist.

2. Verfahren nach Anspruch 1, worin die Präparation eine pharmazeutische oder kosmetische Präparation ist.

3. Verfahren nach Anspruch 1 oder 2, worin das Salz ein Natriumsalz ist.

4. Verfahren nach einem der vorhergehenden Ansprüche, worin R Methyl, 1-Butyl oder 2-Hydroxyethyl ist.

5. Wasser-enthaltende örtlich anwendbare Präparation für Säugetiere, die 3—30 Gew.-% eines Alkalimetallsalzes einer Verbindung der Formel:

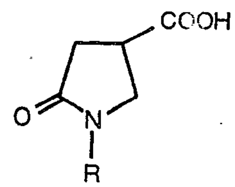

enthält, worin R ($C_1$—$C_5$)-Alkyl oder ($C_1$—$C_5$)-Hydroxyalkyl ist, mit der Maßgabe, daß die Hydroxygruppe nicht an das dem Stickstoffatom benachbarte Kohlenstoffatom substituiert ist.

6. Präparation nach Anspruch 5, die eine pharmazeutische oder kosmetische Präparation ist.

7. Präparation nach Anspruch 5 oder 6, worin das Salz ein Natriumsalz ist.

8. Präparation nach einem der Ansprüche 5 bis 7, worin R Methyl, 1-Butyl oder 2-Hydroxyethyl ist.

9. Alkalimetallsalz einer Verbindung der Formel:

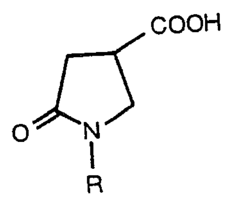

worin R ($C_1$—$C_5$)-Alkyl oder ($C_1$—$C_5$)-Hydroxyalkyl ist, mit der Maßgabe, daß die Hydroxygruppe nicht an einem dem Stickstoffatom benachbarten Kohlenstoffatom ist.

10. Salz nach Anspruch 9, das ein Natriumsalz ist.

**Patentansprüche für die Vertragsstaaten: AT**

1. Verfahren zum Minimieren des Wasserverlusts von einer Wasser-enthaltenden örtlich anwendbaren Präparation für Säugetiere, die Schutz gegenüber Wasserverlust benötigt, das das Einarbeiten in die Präparation von 3—30 Gew.% eines Alkalimetallsalzes einer Verbindung der Formel:

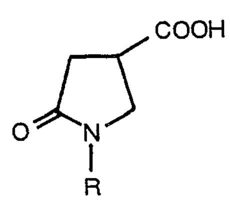

umfaßt, worin R ($C_1$—$C_5$)-Alkyl oder ($C_1$—$C_5$)-Hydroxyalkyl ist, mit der Maßgabe, daß die Hydroxygruppe nicht an einem dem Stickstoffatom benachbarten Kohlenstoffatom ist.

2. Verfahren nach Anspruch 1, worin die Präparation eine pharmazeutische oder kosmetische Präparation ist.

3. Verfahren nach Anspruch 1 oder 2, worin das Salz ein Natriumsalz ist.

4. Verfahren nach einem der vorhergehenden Ansprüche, worin R Methyl, 1-Butyl oder 2-Hydroxyethyl ist.

5. Verfahren zur Herstellung einer Wasser-enthaltenden örtlich anwendbaren Präparation für Säugetiere, das das Formulieren einer wässrigen örtlich anwendbaren Zusammensetzung, die 3—30 Gew.-% eines Alkalimetallsalzes einer Verbindung der Formel:

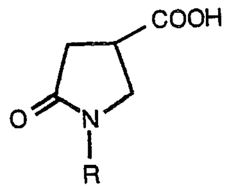

enthält, umfaßt, worin R ($C_1$—$C_5$)-Alkyl oder ($C_1$—$C_5$)-Hydroxyalkyl ist, mit der Maßgabe, daß die Hydroxygruppe nicht an das dem Stickstoffatom benachbarte Kohlenstoffatom substituiert ist.

6. Präparation nach Anspruch 5, das die Herstellung einer pharmazeutischen oder kosmetischen Präparation umfaßt.

7. Verfahren nach Anspruch 5 oder 6, worin das Salz ein Natriumsalz ist.

8. Verfahren nach einem der Ansprüche 5 bis 7, worin R Methyl, 1-Butyl oder 2-Hydroxyethyl ist.

9. Verfahren zur Herstellung eines Alkalimetallsalzes einer Verbindung der Formel:

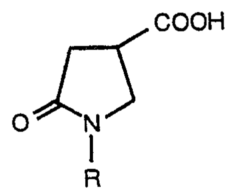

worin R ($C_1$—$C_5$)-Alkyl oder ($C_1$—$C_5$)-Hydroxyalkyl ist, mit der Maßgabe, daß die Hydroxygruppe nicht an einem dem Stickstoffatom benachbarten Kohlenstoffatom ist, das das Umsetzen der entsprechenden freien Säure mit einer Alkalimetallbase umfaßt.

10. Verfahren nach Anspruch 9, worin das Salz ein Natriumsalz ist.

**Revendications pour l'Etat contractant: DE CH DE FR GB IT LI LU NL SE**

1. Procédé pour minimiser la perte d'eau d'une préparation topique contenant de l'eau, destinée à des mammifères, qui nécessite d'être protégée de ladite parte d'eau, qui consiste à incorporer à ladite préparation 3 à 30% en poids d'un sel de métal alcalin d'un composé de formule:

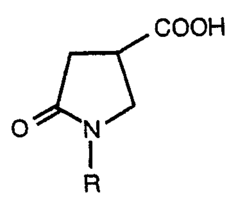

dans laquelle R est un groupe alkyle en $C_1$ à $C_5$ ou hydroxyalkyle en $C_1$ à $C_5$, sous réserve que le groupe hydroxy ne se trouve pas sur un atome de carbone adjacent à l'atome d'azote.

2. Procédé suivant la revendication 1, dans lequel la préparation est une préparation pharmaceutique ou cosmétique.

3. Procédé suivant la revendication 1 ou 2, dans lequel le sel est un sel de sodium.

4. Procédé suivant l'une quelconque des revendications précédentes, dans lequel R est un groupe méthyle, 1-butyle ou 2-hydroxyéthyle.

5. Préparation topique contenant de l'eau, destinée à des mammifères, qui comprend 3—30% en poids d'un sel de métal alcalin d'un composé de formule

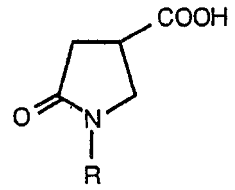

dans laquelle R est un group alkyle en $C_1$ à $C_5$ ou hydroxyalkyle en $C_1$ à $C_5$, sous réserve que le groupe hydroxy ne soit pas substitué sur l'atome de carbone adjacent à l'atome d'azote.

6. Préparation suivant la revendication 5, qui est une préparation pharmaceutique ou cosmétique.

7. Préparation suivant la revendication 5 ou 6, dans laquelle le sel est un sel de sodium.

8. Préparation suivant l'une quelconque des revendications 5 à 7, dans laquelle R est un groupe méthyle, 1-butyle ou 2-hydroxyéthyle.

9. Sel de métal alcalin d'un composé de formule:

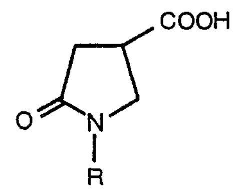

dans laquelle R ést un groupe alkyle en $C_1$ à $C_5$ ou hydroxyalkyle en $C_1$ à $C_5$, sous réserve que le groupe hydroxy ne se trouve pas sur un atome de carbone adjacent à l'atome d'azote.

10. Sel suivant la revendication 9, qui est un sel de sodium.

**Revendications pour l'Etat contractant: AT**

1. Procédé pour minimiser la perte d'eau d'une préparation topique contenant de l'eau, destinée à des mammifères, qui nécessite d'être protégée de ladite parte d'eau, qui consiste à incorporer à préparation 3 à 30% en poids d'un sel de métal alcalin d'un composé de formule:

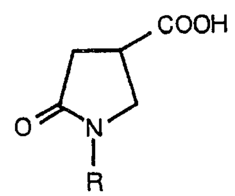

dans laquelle R est un groupe alkyle en $C_1$ à $C_5$ ou hydroxyalkyle en $C_1$ à $C_5$, sous réserve que le groupe hydroxy ne se trouve pas sur un atome de carbone adjacent à l'atome d'azote.

2. Procédé suivant la revendication 1, dans lequel la préparation est une préparation pharmaceutique ou cosmétique.

3. Procédé suivant la revendication 1 ou 2, dans lequel le sel est un sel de sodium.

4. Procédé suivant l'une quelconque des revendications précédentes, dans lequel R est un groupe méthyle, 1-butyle ou 2-hydroxyéthyle.

5. Procédé pour obtenir une préparation topique contenant de l'eau, destinée à des mammifères, qui consiste à formuler une composition aqueuse topique comprenant 3 à 30% en poids d'un sel de métal alcalin d'un composé de formule:

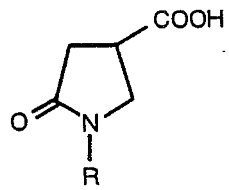

dans laquelle R est un group alkyle en $C_1$ à $C_5$ ou hydroxyalkyle en $C_1$ à $C_5$, sous réserve que le groupe hydroxy ne soit pas substitué sur l'atome de carbone adjacent à l'atome d'azote.

6. Procédé suivant la revendication 5, qui consiste à former une préparation pharmaceutique ou cosmétique.

7. Procédé suivant la revendication 5 ou 6, dans lequel le sel est un sel de sodium.

8. Procédé suivant l'une quelconque des revendications 5 à 7, dans lequel R est un groupe méthyle, 1-butyle ou 2-hydroxyéthyle.

9. Procédé de préparation d'un sel de métal alcalin d'un composé de formule:

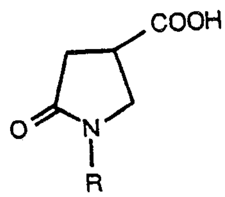

dans laquelle R est un groupe alkyle en $C_1$ à $C_5$ ou hydroxyalkyle en $C_1$ à $C_5$, sous réserve que le groupe hydroxy ne se trouve pas sur un atome de carbone adjacent à l'atome d'azote, qui consiste à faire réagir l'acide libre correspondant avec une base de métal alcalin.

10. Procédé suivant la revendication 9, dans lequel le sel est un sel de sodium.